# EUROPÄISCHE PATENTSCHRIFT

(11) **EP 0 565 216 B1**
(45) Veröffentlichungstag und Bekanntmachung des Hinweises auf die Patenterteilung: **13.01.1999**
(21) Anmeldenummer: 93250099.4
(22) Anmeldetag: 02.04.1993
(51) Int. Cl.: A61B 17/58

(54) **Marknagel**
Medullar nail
Clou médullaire

(30) Priorität: 10.04.1992 DE 9205200 U; 27.10.1992 DE 9214970 U
(43) Veröffentlichungstag der Anmeldung: 13.10.1993
(73) Patentinhaber: aap Implantate AG, 12099 Berlin (DE)
(72) Erfinder: Ahrens, Uwe, W-1000 Berlin 37 (DE)
(74) Vertreter: Meissner, Peter E., Dipl.-Ing.

(56) Entgegenhaltungen:
- EP-A- 0 170 787
- US-A- 4 574 795

## Beschreibung

Die Erfindung betrifft einen Marknagel, an dem mehrere Ausnehmungen vorgesehen sind, die zusammen mit Schrauben eine Verriegelung des Nagels im Knochen ermöglichen, sowie mit einer Halteanordnung am, proximalen Ende des Nagels.

Derartige Marknägel werden verwendet bei allen) röhrenartigen Knochen von Menschen und Tieren zur Frakturheilung bzw. Knochenverlängerung.

Bei bisher aus der Praxis bekannten Marknägeln erfolgte die Verriegelung im Knochen mittels Schrauben, die durch Bohrungen in dem Marknagel hindurchgeführt werden. Am proximalen Ende des Nagels war bei den bekannte Marknägeln ein Langloch vorgesehen, das mit einem Haken zusammen zum Ausschlagen verwendet werden konnte.

Probleme ergaben sich einerseits aus der Notwendigkeit, die Schrauben durch die Bohrungen mit dem Nagel hindurchzuführen, und darüber hinaus war die Verbindung des Marknagels mit dem Haken umständlich.

Aus der 0 170 787 A1 ist eine Vorrichtung zum Fixieren von Röhrenknochenfrakturen bekannt, bei der das Problem, den zu setzenden Marknagel beim Einbringen in den Knochen in eine bestimmte Position zu führen, gelöst werden soll.

Zu diesem Zweck ist der Marknagel in seinem vorderen Teil mit einer schräg nach vorn offenen Auflaufnut versehen (s. Fig. 9 und 10).

Es wird zunächst ein Führungsbolzen quer zur Knochenlängsachse gesetzt, auf den sodann die im Spitzenbereich des Marknagels angeordnete Auflaufnut durch Axialverschiebung des Nagels aufgeschoben wird.

Die endgültige Fixierung des Nagels vollzieht sich durch Bolzen, die durch Löcher im Nagel geführt werden und unterliegt damit der großen Schwierigkeit, die Löcher im Nagel mit den Bolzen zu treffen. Bei mit Röntgenstrahlung arbeitenden Zielvorrichtungen ergibt sich eine hohe Strahlenbelastung für den Patienten.

Die nach außen offene Auflaufnut kann wegen ihrer Erstreckung schräg zur Nagellängsachse keinen Beitrag zur Fixierung eines Knochenbruches leisten, da der Nagel durch den in diese Nut eingelegten Bolzen nicht an seiner Axialbewegung entgegen der Einsetzbewegung gehindert wird.

Der Erfindung lag die Aufgabe zugrunde, einen Marknagel zu schaffen, der in der Handhabung einfach ist, verbesserte Festigkeitseigenschaften aufweist und im Knochen gegen achsiale Verschiebung und Torsion gesichert ist.

Gelöst wird diese Aufgabe erfindungsgemäß entsprechend dem Merkmalen des Patentanspruchs 1. Die Unteransprüche geben zweckmäßige Ausgestaltungen wieder. Anstelle der Ausnehmungen in Form von Bohrungen für die Schrauben sind erfindungsgemäß Kerben auf gegenüberliegenden Seiten des Nagels vorgesehen, so daß die Schrauben lediglich beim Verriegeln in diese Kerben eingesetzt werden müssen. Die Ausbildung der Kerben, insbesondere bei abgerundeten Kanten der Kerben, ergibt eine wesentliche Spannungsoptimierung, die sich insgesamt auf die Festigkeit des Nagels und die Verriegelung positiv auswirkt.

Schließlich bieten die Gewinde am proximalen Ende des Nagels eine hohe Sicherheit bei der Entfernung des Nagels und gegenüber den herkömmlichen Lösungen ist der Einsatz wesentlich vereinfacht.

Eine weitere Verbesserung wird dadurch erreicht, daß die Kerben derart ausgebildet sind, daß mit den zur Verriegelung dienenden Schrauben eine kraft- und/oder formschlüssige Verbindung besteht. Im einzelnen kann diese Verbindung so gestaltet sein, daß die Längsachse der Kerbe unter einem bestimmten Winkel zur Längsachse des Nagels verläuft und daß die Winkel nebeneinander liegender Kerben unterschiedlich sind. Fügt man nun in diese Kerben die Schrauben ein, so bilden sie mit dem Nagel beispielsweise je nach Ausbildung des Winkels ein Dreieck oder ein offenes Viereck. Eine weitere Variationsmöglichkeit kann darin bestehen, daß die Kerben mit einem Gewinde versehen sind, die in Eingriff gelangen mit dem Gewinde an den Schrauben. Schließlich können auch die Kerben am oberen Rand Hinterschneidung aufweisen, die die Schraubenoberfläche im Querschnitt gesehen teilweise umfassen. Mit dieser erfindungsgemäßen Ausbildung wird ein seitliches Verrutschen des Nagels in den Epiphysen des Knochens verhindert. Bekanntlich weist der Markraum des Röhrenknochens im mittleren Bereich, also der Diaphyse, den geringsten Durchmesser auf, und dieser ist für die Nagelauswahl bei der Verriegelungsnagelung ohne Aufbohren des Markraumes ausschlaggebend. Zu den Epiphysen, also den Enden des Knochens hin, wird der Markraum größer. Durch die form- und/oder kraftschlüssige Verbindung zwischen dem Nagel und den Schrauben wird ein Verschieben des Nagels in der Vertikalebene verhindert. Allein bei Einsatz von zwei Schrauben am Nagel wird bereits eine Kippstabilität erreicht, da jede Schraube ein mögliches Drehzentrum in einer Richtung darstellt und damit das Kippen in der Sagittalebene verhindert wird.

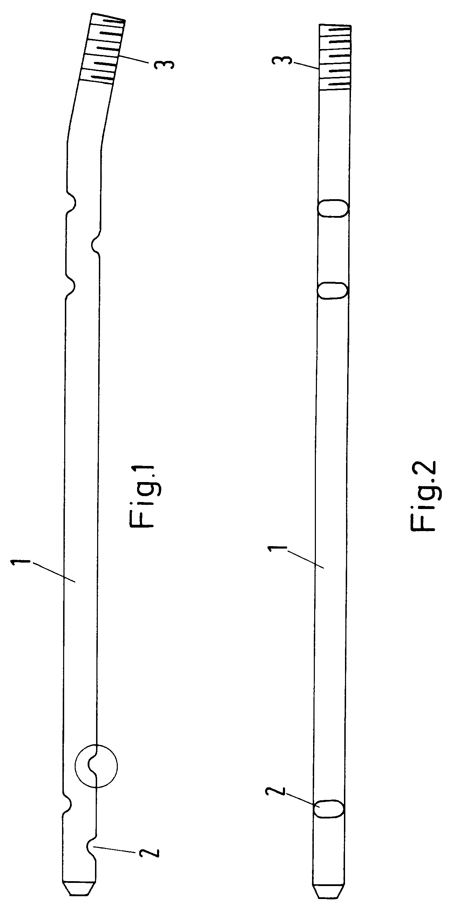

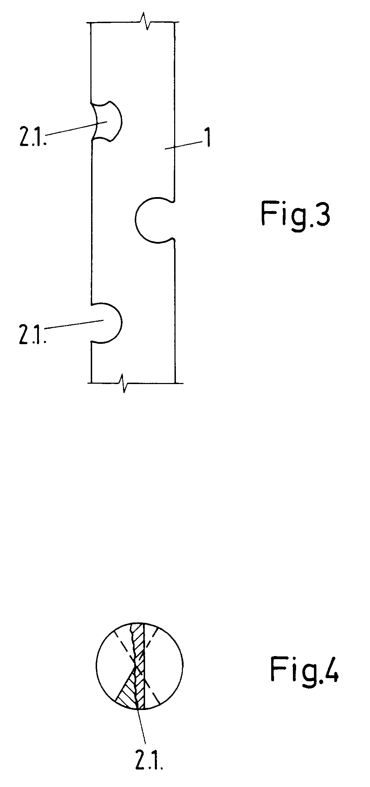

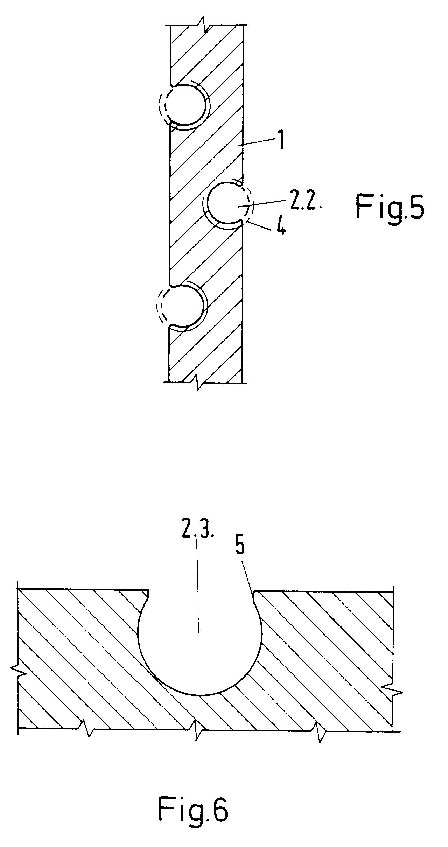

Die Erfindung soll anhand der Figuren 1 bis 6 erläutert werden, wobei diese in Seitenansicht bzw. -aufsicht oder Teilschnitt den Marknagel zeigen.

Am einen Ende des Marknagels 1 (Figur 1) sind die Kerben 2 zu erkennen mit den abgerundeten Kanten. Im vorliegenden Beispiel sind auf der einen Seite zwei Kerben vorgesehen und gegenüberliegend eine dritte. Ähnliche Kerben können auch am proximalen Ende des Nagels vorhanden sein, wie sich aus der Zeichnung ergibt.
Ferner ist am proximalen Ende des Nagels ein Gewinde 3 zu erkennen. Zweckmäßigerweise ist dieses Gewinde als Innen- und Außengewinde ausgebildet, so daß für das Ausschlagen beide Gewinde benutzbar sind.

Die Anzahl der Kerben kann beliebig gewählt werden, wobei die Anordnung sich gegenüberliegender Kerben für die Torsionssicherung vorteilhaft ist. Zusätzlich können allerdings im Nagel auch noch Bohrungen angeordnet sein für die Anbringung einer Torrionssicherung. Der Nagel kann darüber hinaus zumindest auf einem Teil seiner axialen Erstreckung an einer Seite abgeflacht sein. Hierdurch werden Zerstörungen der Gefäße vermieden, die im Knochen zur Knochenernährung vorhanden sind.

Besonderheiten der Kerbenausbildung ergeben sich aus den Figuren 3 bis 6. Dabei ist in Figur 3 dargestellt, daß die Kerben unter einem Winkel zur Längsachse des Nagels 1 ausgebildet sind. Diese Kerben sind hier in den Figuren 3 und 4 mit 2.1. bezeichnet . Eine weitere Möglichkeit ist in Figur 5 dargestellt, wo in die Kerben 2.2. eine Gewinde 4 eingeschnitten ist, und schließlich ist in Figur 6 vereinfacht dargestellt, daß die Kerben 2.3. Hinterschneidungen 5 aufweisen, die die Schrauben zumindest teilweise auf ihrem Umfang umfassen, dennoch aber ein einfaches Einfügen ermöglichen.

## Patentansprüche

1. Marknagel mit Schrauben zur Verriegelung des Nagels im Knochen, der mit mehreren Ausnehmungen versehen ist, in die die Schrauben einsetzbar sind und der am proximalen Ende mit einer Halteanordnung für das Einsetzen bzw.Extrahieren des Nagels verbindbar ist, wobei die Ausnehmungen als seitlich von der Längsachse des Marknagels auf gegenüberliegenden Seiten angeordnete Kerben (2) ausgebildet sind, wobei die Karben (2) derart gestaltet sind, daß die Schrauben nur in Richtung der Längsachse der Kerben einsetzbar sind so daß zwischen dem Nagel (1) und den Schrauben eine kraft- und/oder formschlüssige Verbindung besteht, wobei die Längsachse einer Kerbe(2) in einem bestimmten Winkel zur Längsachse des Marknagels (1) verläuft und wobei zur Befestigung der Halteanordnung am proximalen Ende des Marknagels ein Außen-und ein Innengewinde (3) vorgesehen sind.

2. Marknagel nach Anspruch 1,
dadurch gekennzeichnet,
daß die Längsachsen der Kerben (2) in Bezug auf den Querschnitt des Nagels (1) sekantenartig verlaufen.

3. Marknagel nach den Ansprüchen 1 und 2,
dadurch gekennzeichnet,
daß die Kerben (2) V-förmig ausgebildet sind.

4. Marknagel nach den Ansprüchen 1 und 2,
dadurch gekennzeichnet,
daß die Kerben (2) einen halbkreisförmigen Querschnitt aufweisen.

5. Marknagel nach Anspruch 1 und 2,
dadurch gekennzeichnet,
daß die Kerben am oberen Rand Hinterschneidungen (5) aufweisen, die die Schraubenoberfläche (im Querschnitt gesehen) teilweise umfassen.

6. Marknagel nach einem der Ansprüche 3 bis 5,
dadurch gekennzeichnet,
daß die Kanten der Kerben (2) abgerundet sind.

7. Marknagel nach einem der Ansprüche 3 bis 5,
dadurch gekennzeichnet,
daß in die Kerben ein Gewinde (4) eingeschnitten ist, dem ein entsprechendes Gewinde an den Schrauben zugeordnet ist.

8. Marknagel nach einem der vorstehenden Ansprüche 1 bis 7,
dadurch gekennzeichnet,
daß der Nagel zumindest auf einem Teil seiner axialen Erstreckung abgeflacht ist.

## Claims

1. Medullary nail with screws for locking the nail in the bone, which nail is provided with a plurality of recesses in which the screws can be inserted and can be connected at the proximal end to a holding arrangement for inserting or extracting the nail, wherein the recesses are formed as notches (2) disposed laterally of the longitudinal axis of the medullary nail on opposite sides, wherein the notches (2) are formed such that the screws can only be inserted in the direction of the longitudinal axis of the notches so that there is a non-positive and/or positive connection between the nail (1) and the screws, wherein the longitudinal axis of a notch (2) extends at a certain angle to the longitudinal axis of the medullary nail (1), and wherein an external and an internal thread (3) are provided for fastening the holding arrangement to the proximal end of the medullary nail.

2. Medullary nail according to claim 1,
characterised in that the longitudinal axes of the notches (2) extend like secants in relation to the cross section of the nail (1).

3. Medullary nail according to claims 1 and 2,
characterised in that the notches (2) are V-shaped.

4. Medullary nail according to claims 1 and 2,
characterised in that the notches (2) have a semicircular cross section.

5. Medullary nail according to claims 1 and 2,
characterised in that the notches at the top edge comprise undercuts (5) which partly embrace the screw surface (viewed in cross section).

6. Medullary nail according to one of claims 3 to 5,
characterised in that the edges of the notches (2) are rounded.

7. Medullary nail according to one of claims 3 to 5,
characterised in that a thread (4) is cut into the notches, with which thread a corresponding thread at the screws is associated.

8. Medullary nail according to one of the preceding claims 1 to 7,
characterised in that the nail is flattened at least over a part of its axial extent.

## Revendications

1. Clou médullaire ayant des vis pour le verrouillage du clou dans l'os, qui est muni de plusieurs évidements dans lesquels les vis peuvent être mises en place et qui, à l'extrémité proximale, peut être relié à un dispositif de maintien pour la mise en place ou l'extraction du clou, les évidements étant réalisés sous forme d'entailles (2) agencées sur des côtés opposés latéralement à l'axe longitudinal du clou médullaire, les entailles (2) étant telles que les vis ne peuvent être introduites qu'en direction de l'axe longitudinal des entailles, de sorte que, entre le clou (1) et les vis, il existe une liaison par coopération de formes et/ou sous l'action d'une force, l'axe longitudinal d'une entaille (2) s'étendant sous un angle déterminé par rapport à l'axe longitudinal du clou médullaire (1) et un filetage externe et un filetage interne (3) étant prévus pour la fixation du dispositif de maintien à l'extrémité proximale du clou médullaire.

2. Clou médullaire selon la revendication 1,
caractérisé en ce que les axes longitudinaux des entailles (2) s'étendent à la manière de sécantes relativement à la section transversale du clou (1).

3. Clou médullaire selon les revendications 1 et 2,
caractérisé en ce que les entailles (2) sont réalisées en forme de V.

4. Clou médullaire selon les revendications 1 et 2,
caractérisé en ce que les entailles (2) présentent une section transversale semi-circulaire.

5. Clou médullaire selon les revendications 1 et 2,
caractérisé en ce que les entailles présentent, sur le bord supérieur, des contre-dépouilles (5) qui entourent partiellement la surface supérieure des vis (en coupe transversale).

6. Clou médullaire selon une des revendications 3 à 5,
caractérisé en ce que les arêtes des entailles (2) sont arrondies.

7. Clou médullaire selon une des revendications 3 à 5,
caractérisé en ce que, dans les entailles, il est usiné un filetage (4) auquel est associé un filetage correspondant sur les vis.

8. Clou médullaire selon une des revendications précédentes 1 à 7,
caractérisé en ce que le clou est aplati au moins sur une partie de son extension axiale.
